# EUROPEAN PATENT SPECIFICATION

(11) **EP 2 694 538 B1**
(45) Date of publication and mention of the grant of the patent: **14.09.2016**
(21) Application number: 12713969.9
(22) Date of filing: 05.04.2012
(51) Int. Cl.: C07K 14/47, A61K 38/17, A61K 38/49, A61K 45/06, A61K 31/4365, A61K 31/616

(54) **THERAPEUTIC AND PROPHYLACTIC METHODS, USES AND COMPOSITIONS COMPRISING ANEXIN A5**
THERAPEUTISCHE UND PROPHYLAKTISCHE VERFAHREN, ANWENDUNGEN UND ZUSAMMENSETZUNGEN MIT ANEXIN A5
MÉTHODES THÉRAPEUTIQUES ET PROPHYLACTIQUES, UTILISATIONS ET COMPOSITIONS COMPORTANT DE L'ANNEXINE A5

(30) Priority: 05.04.2011 US 201161472002 P; 05.04.2011 US 201161472013 P
(43) Date of publication of application: 12.02.2014
(73) Proprietor: Annexin Pharmaceuticals AB, 247 26 Veberöd (SE)
(72) Inventor: PETTERSSON, Knut, 414 76 Göteborg (SE)
(74) Representative: Potter Clarkson LLP
(86) International application number: PCT/EP2012/056370
(87) International publication number: WO 2012/136819

(56) References cited:
- WO-A1-2009/103977
- TEOH ET AL: "Diannexin, a Novel Annexin V Homodimer, Provides Prolonged Protection Against Hepatic Ischemia-Reperfusion Injury in Mice", GASTROENTEROLOGY, ELSEVIER, PHILADELPHIA, PA, vol. 133, no. 2, 3 August 2007 (2007-08-03), pages 632-646, XP022198888, ISSN: 0016-5085, DOI: 10.1053/J.GASTRO.2007.05.027 cited in the application
- MOLSKI MICHAL ET AL: "Diannexin treatment decreases ischemia-reperfusion injury at the endothelial cell level of the microvascular bed in muscle flaps.", ANNALS OF PLASTIC SURGERY NOV 2009 LNKD- PUBMED:19806047, vol. 63, no. 5, November 2009 (2009-11), pages 564-571, XP008153086, ISSN: 1536-3708
- CEDERHOLM ANNA ET AL: "Annexin A5 as a novel player in prevention of atherothrombosis in SLE and in the general population", NEW YORK ACADEMY OF SCIENCES. ANNALS, WILEY-BLACKWELL PUBLISHING, INC, US, vol. 1108, 1 June 2007 (2007-06-01), pages 96-103, XP008107390, ISSN: 0077-8923, DOI: 10.1196/ANNALS.1422.011 [retrieved on 2007-08-29] cited in the application
- KIMBERLEY E. WEVER ET AL: "Diannexin Protects against Renal Ischemia Reperfusion Injury and Targets Phosphatidylserines in Ischemic Tissue", PLOS ONE, vol. 6, no. 8, 30 August 2011 (2011-08-30) , page E24276, XP55031079, ISSN: 1932-6203, DOI: 10.1371/journal.pone.0024276
- K. J. Kelly: "Distant Effects of Experimental Renal Ischemia/Reperfusion Injury", Journal of the American Society of Nephrology, vol. 14, no. 6, 1 June 2003 (2003-06-01), pages 1549-1558, XP055133279, ISSN: 1046-6673, DOI: 10.1097/01.ASN.0000064946.94590.46

### Sequence listing

<110> Athera Biotechnologies AB
<110> Knut Pettersson
<120> Therapeutic and Prophylactic Methods, Uses and Compositions
<130> ATHBE/P50909PC
<150> US 61/472013
   <151> 2011-04-05
<150> US 61/472002
   <151> 2011-04-05
<160> 1
<170> PatentIn version 3.3
<210> 1
   <211> 320
   <212> PRT
   <213> Homo sapiens
<220>
   <221> misc_feature
   <223> Human Annexin A5
<400> 1
   Met Ala Gln Val Leu Arg Gly Thr Val Thr Asp Phe Pro Gly Phe Asp
   1               5                   10                  15
   Glu Arg Ala Asp Ala Glu Thr Leu Arg Lys Ala Met Lys Gly Leu Gly
               20                  25                  30
   Thr Asp Glu Glu Ser Ile Leu Thr Leu Leu Thr Ser Arg Ser Asn Ala
           35                  40                  45
   Gln Arg Gln Glu Ile Ser Ala Ala Phe Lys Thr Leu Phe Gly Arg Asp
       50                  55                  60
   Leu Leu Asp Asp Leu Lys Ser Glu Leu Thr Gly Lys Phe Glu Lys Leu
   65                  70                  75                  80
   Ile Val Ala Leu Met Lys Pro Ser Arg Leu Tyr Asp Ala Tyr Glu Leu
                   85                  90                  95
   Lys His Ala Leu Lys Gly Ala Gly Thr Asn Glu Lys Val Leu Thr Glu
               100                 105                 110
   Ile Ile Ala Ser Arg Thr Pro Glu Glu Leu Arg Ala Ile Lys Gln Val
           115                 120                 125
   Tyr Glu Glu Glu Tyr Gly Ser Ser Leu Glu Asp Asp Val Val Gly Asp
       130                 135                 140
   Thr Ser Gly Tyr Tyr Gln Arg Met Leu Val Val Leu Leu Gln Ala Asn
   145                 150                 155                 160
   Arg Asp Pro Asp Ala Gly Ile Asp Glu Ala Gln Val Glu Gln Asp Ala
                   165                 170                 175
   Gln Ala Leu Phe Gln Ala Gly Glu Leu Lys Trp Gly Thr Asp Glu Glu
               180                 185                 190
   Lys Phe Ile Thr Ile Phe Gly Thr Arg Ser Val Ser His Leu Arg Lys
           195                 200                 205
   Val Phe Asp Lys Tyr Met Thr Ile Ser Gly Phe Gln Ile Glu Glu Thr
       210                 215                 220
   Ile Asp Arg Glu Thr Ser Gly Asn Leu Glu Gln Leu Leu Leu Ala Val
   225                 230                 235                 240
   Val Lys Ser Ile Arg Ser Ile Pro Ala Tyr Leu Ala Glu Thr Leu Tyr
                   245                 250                 255
   Tyr Ala Met Lys Gly Ala Gly Thr Asp Asp His Thr Leu Ile Arg Val
               260                 265                 270
   Met Val Ser Arg Ser Glu Ile Asp Leu Phe Asn Ile Arg Lys Glu Phe
           275                 280                 285
   Arg Lys Asn Phe Ala Thr Ser Leu Tyr Ser Met Ile Lys Gly Asp Thr
       290                 295                 300
   Ser Gly Asp Tyr Lys Lys Ala Leu Leu Leu Leu Cys Gly Glu Asp Asp
   305                 310                 315                 320

## Description

### Field of the Invention

The invention relates to novel uses and compositions for use in the prevention and/or reduction of peri- and postoperative complications following surgical intervention, such as complications following vascular surgery, especially peripheral vascular surgery.

### Background to the Invention

The listing or discussion of an apparently prior-published document in this specification should not necessarily be taken as an acknowledgement that the document is part of the state of the art or is common general knowledge.

It is well known that surgical interventions frequently lead to complications. Not surprisingly, such complications are more common after major and long lasting interventions than after rapid and simple interventions. The status of the patient is also important for the risk of complications. Interventions normally require some form of anaesthesia, and the anaesthesia itself can lead to complications.

Peri- and post-operative complications can affect the organ or organs that are the target for the intervention, but can also affect other organs. One example is the risk for deep vein thrombosis that can be a result of orthopaedic surgery such as hip replacement surgery (Duggan. Am J Cardiovasc Drugs, 2012. 12(1): p. 57-72). To make surgery easier or even possible, it is common to stop blood flow to the target organ during the intervention. This can result in target organ injury, but also in complications in other organs. An example of this is lung injury following a blockade of intestinal blood flow (Feinman. Am J Physiol Gastrointest Liver Physiol, 2010. 299(4): p. G833-43). The mechanical trauma to a target organ during surgery can lead to cell death and direct injury to the organ, but this can also result in damage to distant organs. One such example is the release of myoglobin from skeletal muscle being injured during surgical intervention that can lead to post surgical kidney injury.

The complications of surgical interventions range in severity from minor to very serious complication, such as death. Post surgical complications also range from very frequent to very infrequent. Examples of frequent complications are complications following interventions aimed at to restoring blood flow in limbs in patients with critical limb ischemia due to peripheral artery disease which lead to death or amputation in up to 30 % of the patients within one month after surgery. In contrast, pituitary infarction following cardiac bypass surgery, which is a common form of surgery, has been described in only 4 patients (Zayour and Azar. Endocr Pract, 2006. 12(1): p. 59-62).

From the above, it becomes evident that preventive treatment that reduces the risk and incidence of peri- and post-operative complications following surgery, especially vascular surgery is at great need.

Annexin A5 is an endogenous protein that binds to charged phospholipids such as phosphatidylserine (PS) (Cederholm and Frostegard (2007) Ann. NY Acad. Sci., 1108:96-103). Annexin A5 is a potent anti-thrombotic agent (Thiagarajan and Benedict (1997) Circulation, 96(7):2339-2347), and it is proposed that Annexin A5 by binding to exposed PS can form a 'protective shield' that can inhibit the effects of PS on thrombosis formation (Rand (2000) Autoimmun., 15(2):107-111).

It has been shown that in addition to anti-platelet and anti-coagulant effects of Annexin A5, this protein and an analogue thereof, the Annexin A5 dimer diannexin, is effective in preventing reperfusion injury in the liver, and it improved the outcome of rat liver transplants (Shen et al. (2007) Am. J. Transplant., 7(11):2463-2471.). Interestingly, in these studies the treatments were associated with a reduced inflammatory activity in the hepatic endothelium, measured as reduced expression of adhesion molecules, that is Annexin A5 has anti-inflammatory effects. It was suggested that diannexin improved the survival of the liver transplants by an anti-thrombotic effect leading to maintained blood supply to the liver (Shen et al. (2007) Am. J. Transplant., 7(11):2463-2471).

It has earlier been suggested that Annexin A5 can be used to stabilise atherosclerotic lesions in coronary arteries in patients, which should reduce the risk for myocardial infarction in these patients (WO 2005/099744).

Annexin A5 has further been suggested as a treatment of various vascular dysfunctions such as angina pectoris (WO 2009/077764) and for the prevention of restenosis (WO 2009/103977).

### Summary of the invention

The invention is defined in the accompanying claims.

Disclosed herein is a method for the prevention and/or the reduction of peri- and postoperative complications following surgical interventions comprising administering a therapeutically effective amount of Annexin A5 or a functional analogue or variant thereof to a patient in need of such treatment.

The first aspect of the invention provides a pharmaceutical composition comprising a therapeutically effective amount of Annexin A5 or a functional analogue or variant thereof for use in the prevention and/or the reduction of peri- and postoperative complications following surgical intervention,
wherein the surgical intervention comprises restrictions in organ blood flow during the surgical intervention, and further wherein the surgical intervention causes immediate cellular damage, ischemia and/or reperfusion injury to a target organ,
wherein the peri- or postoperative complication is a complication of an organ not being the target of the surgical intervention, and
wherein the Annexin A5 or the functional analogue or variant thereof is selected from:
   (a) human Annexin A5 (SEQ ID NO:1);
   (b) a mammalian orthologue of human Annexin A5;
   (c) an allelic or genetic variant of a) or b);
   (d) a functional analogue of Annexin which is a protein which is more than 90%, or preferably more than 95% identical to human Annexin A5, SEQ ID NO:1;
   (e) a dimer of, or a fusion protein comprising, any of a), b), c) or d); and
   (f) a PEGylated variant of any of a), b), c), d) or e).

Put another way, the first aspect of the invention also provides for the use of Annexin A5 or a functional analogue or variant thereof in the manufacture of a medicament for the prevention and/or the reduction of peri- and postoperative complications following surgical intervention, wherein the surgical intervention comprises restrictions in organ blood flow during the surgical intervention, and further wherein the surgical intervention causes immediate cellular damage, ischemia and/or reperfusion injury to a target organ,
wherein the peri- or postoperative complication is a complication of an organ not being the target of the surgical intervention, and
wherein the Annexin A5 or the functional analogue or variant thereof is selected from:
   (a) human Annexin A5 (SEQ ID NO:1);
   (b) a mammalian orthologue of human Annexin A5;
   (c) an allelic or genetic variant of a) or b);
   (d) a functional analogue of Annexin which is a protein which is more than 90%, or preferably more than 95% identical to human Annexin A5, SEQ ID NO:1;
   (e) a dimer of, or a fusion protein comprising, any of a), b), c) or d); and
   (f) a PEGylated variant of any of a), b), c), d) or e).

It is contemplated that any use or composition described herein can be implemented with respect to any other use or composition described herein.

The use of the word "a" or "an" when used in conjunction with the term "comprising" in the claims and/or the specification may mean "one," but it is also consistent with the meaning of "one or more," "at least one," and "one or more than one."

These, and other, embodiments of the invention will be better appreciated and understood when considered in conjunction with the following description and the accompanying drawings. It should be understood, however, that the following description, while indicating various embodiments of the invention and numerous specific details thereof, is given by way of illustration and not of limitation. Many substitutions, modifications, additions and/or rearrangements may be made within the scope of the invention without departing from the spirit thereof, and the invention includes all such substitutions, modifications, additions and/or rearrangements.

### Description of the figures

Figure 1 shows changes in LDH during ischemia and reperfusion in an in vivo rat model, with or without Annexin A5 pre-treatment, as described in Example 1.
**Figure 2** shows changes in lung inflammation following intestinal ischemia in an in vivo rat model, with or without Annexin A5 pre-treatment, as described in Example 2.

### Detailed description of the invention

Here, the examples presented herein are designed to illustrate the utility of Annexin A5 to reduce the occurrence of postoperative complications following surgery, by *in vivo* testing in a rat model. Thus, Annexin A5 or functional analogues and/or variants thereof present a new treatment modality that can reduce peri- and post-operative complications in patients undergoing surgical interventions, such as vascular surgery.

The present application describes a method for the prevention and/or the reduction of peri- and post-operative complications following surgical intervention, said method comprising administering a therapeutically effective amount of Annexin A5 or a functional analogue or variant thereof to a patient in need of such treatment.

The first aspect of the invention provides pharmaceutical composition comprising a therapeutically effective amount of Annexin A5 or a functional analogue or variant thereof for use in the prevention and/or the reduction of peri- and postoperative complications following surgical intervention,
wherein the surgical intervention comprises restrictions in organ blood flow during the surgical intervention, and further wherein the surgical intervention causes immediate cellular damage, ischemia and/or reperfusion injury to a target organ,
wherein the peri- or postoperative complication is a complication of an organ not being the target of the surgical intervention, and
wherein the Annexin A5 or the functional analogue or variant thereof is selected from:
   (a) human Annexin A5 (SEQ ID NO:1);
   (b) a mammalian orthologue of human Annexin A5;
   (c) an allelic or genetic variant of a) or b);
   (d) a functional analogue of Annexin which is a protein which is more than 90%, or preferably more than 95% identical to human Annexin A5, SEQ ID NO:1;
   (e) a dimer of, or a fusion protein comprising, any of a), b), c) or d); and
   (f) a PEGylated variant of any of a), b), c), d) or e).

The first aspect of the invention also provides for the use of Annexin A5 or a functional analogue or variant thereof in the manufacture of a medicament for the prevention and/or the reduction of peri- and postoperative complications following surgical intervention,
wherein the surgical intervention comprises restrictions in organ blood flow during the surgical intervention, and further wherein the surgical intervention causes immediate cellular damage, ischemia and/or reperfusion injury to a target organ,
wherein the peri- or postoperative complication is a complication of an organ not being the target of the surgical intervention, and
wherein the Annexin A5 or the functional analogue or variant thereof is selected from:
   (a) human Annexin A5 (SEQ ID NO:1);
   (b) a mammalian orthologue of human Annexin A5;
   (c) an allelic or genetic variant of a) or b);
   (d) a functional analogue of Annexin which is a protein which is more than 90%, or preferably more than 95% identical to human Annexin A5, SEQ ID NO:1;
   (e) a dimer of, or a fusion protein comprising, any of a), b), c) or d); and
   (f) a PEGylated variant of any of a), b), c), d) or e).

In one embodiment of the first aspect of the present invention, Annexin A5 or a functional analogue or variant thereof is used for peri- or post-operative organ protection. Thus, the patient (preferably, a human patient) is treated to prevent, reduce or ameliorate damage to organs that occurs during or following a surgical intervention, including anaesthesia. The peri- or post-operative complication is reduced in an organ not being the target of the surgical intervention. As such, the organ(s) to be protected are distal from the site of the surgical intervention. In a further preferred embodiment, the organ(s) to be protected are distal from the site of the surgical intervention and are also not directly affected by the surgical procedure. In this context, "directly affected" may include the effect of one, two or all three of ischemia, reperfusion and/or thrombosis as a result of the surgery. Thus, in one embodiment, the organ(s) to be protected by the present invention do not experience any one or more, preferably all, of immediate cellular damage, ischemia, reperfusion and/or thrombosis as a result of the surgical intervention.

For example, Example 1 of the present application relates to the use of Annexin A5 to prevent liver and renal damage following a surgical intervention that causes ischemia and reperfusion in lower limbs; surgical interventions that causes ischemia and reperfusion in lower limbs do not have the effect of causing ischemia and reperfusion in the liver and the kidney, but nevertheless a surgical intervention to a lower limb can cause organ damage in the kidney and elsewhere. The present application describes methods to prevent, reduce or ameliorate such damage that can occur during or following a surgical intervention performed on, or causing ischemia and/or reperfusion in, the lower limbs.

Likewise, Example 2 of the present application relates to the use of Annexin A5 to prevent lung damage following a surgical intervention that causes ischemia and reperfusion in the intestines; surgical interventions that causes ischemia and reperfusion in the intestine do not have the effect of causing ischemia and reperfusion in the lungs, but nevertheless a surgical intervention to the intestines can cause organ damage in the lungs. The present application describes methods to prevent, reduce or ameliorate damage to the lungs that can occur during or following a surgical intervention performed on, or causing ischemia and/or reperfusion in, the intestines.

There are a large number of examples known in the art of surgical interventions in one part of the body causing indirect organ damage in other parts of the body. Prior to the present invention, there was no effective way to prevent the indirect organ damage that followed during, or after, some surgical interventions. If, for example, as a result of a surgical procedure performed on, or causing ischemia and/or reperfusion in, the intestines, the patient survived the initial ischemic event and post-operative indirect complications resulted which caused organ damage to the lungs, then the person skilled in the art would only be able to treat the organ damage to the lungs after the damage had occurred, for example by lung ventilation and/or anti-inflammatory drugs. If, for example, as a result of a surgical procedure performed on, or causing ischemia and/or reperfusion in, a lower limb, the patient survived the initial ischemic event and post-operative indirect complications resulted which caused organ damage to the kidney, then the person skilled in the art would only be able to treat the organ damage to the kidney after the damage had occurred, for example by haemodilution, dialysis or, if necessary, transplantation.

The aim of the present invention is to use Annexin A5 to treat/prevent this type of indirect organ damage following surgery/anaesthesia.

In one embodiment, the type of organ damage to be treated is not thrombosis and/or the downstream effects thereof. Thus, the claims of the present application are not intended to encompass the use of Annexin A5 to treat or prevent thrombosis in any organs.

Thus, in one embodiment, the first aspect of the present invention relates to the use of Annexin A5 or a functional analogue or variant thereof for peri- or postoperative organ protection in order to prevent organ damage in an organ wherein the patient is subjected to a surgical intervention (including anaesthesia) that is at a site that is distal from the organ that is to be protected from damage, and wherein the organ to be protected from damage does not experience one or more (including all) of ischemia, reperfusion and thrombosis, or any other direct effect, as a result of the surgical intervention.

For example, in one embodiment, the patient may be an elderly patient (for example, at an age about, or greater than, 50, 55, 60, 65, 70, 75, 80, 85, 90 or more years old) in whom even anaesthesia can sometimes be enough to cause organ damage (such as myocardial damage and infarction) at a site distal from the site of administration of the anaesthetic. Accordingly, in one aspect of the present invention, Annexin A5 or a functional analogue or variant thereof can be used to prevent organ damage, such as myocardial damage and/or infarction, in an elderly patient who is treated, is to be treated, or has been treated with an anaesthetic.

In another exemplary embodiment, patients suffering from diabetes can experience critical limb ischemia, and can have a higher incidence rate of cardiac events than non-diabetic patients. Accordingly, in another aspect of the present invention, Annexin A5 or a functional analogue or variant thereof can be used to prevent organ damage, such as cardiac damage, in a patient with diabetes who critical limb ischemia, for example when treated by a surgical intervention.

The patient is typically a human patient. Alternatively, the patient may be a non-human animal, such as a domestic animal (for example, cat, dog, rabbit, cow, sheep, pig, mouse or other rodent).

Peri- and postoperative complications can occur after any form of surgical intervention. Different types of surgical interventions where Annexin A5 or a functional analogue or variant thereof can be used to prevent and/or reduce postoperative complications are exemplified by, but not limited to, cardiovascular surgery, such as cardiac surgery and cardiopulmonary bypass, vascular surgery, such as peripheral vascular surgery, pulmonary surgery, digestive system surgery, such as gastrectomy and colectomy, obstetric surgery, such as caesarean sections, orthopaedic surgery, such as joint replacements, especially hip replacements, reconstructive surgery, transplantations, such as kidney transplantations, heart transplantations, liver transplantations and lung transplantations, as well as trauma surgery and amputations.

As discussed above, peri- and postoperative complications are more common after major and long lasting interventions than after rapid and simple interventions. In an embodiment of the first aspect of the present invention the duration of the surgical intervention may be at least, or greater than 5, 10, 15 20, 30, 40, 50, 60, 90, 120, 150, 180, or 240 minutes.

The surgical interventions where Annexin A5 or a functional analogue or variant thereof can be used according to the invention include interventions comprising restrictions in organ blood flow during part (such as, at least or greater than half), or all, of the duration of the intervention.

The surgical interventions where Annexin A5 or a functional analogue or variant thereof can be used according to the invention further include interventions wherein the intervention causes immediate cellular damage (i.e. physical injury) to the target organ.

The peri- and postoperative complications that can be prevented and/or reduced through the uses and/or pharmaceutical compositions according to the present invention are both complications arising in the organ not being the target of the surgical intervention. As discussed above, the surgical intervention (including anaesthesia) is typically at a site that is distal from the target organ that is to be protected from peri- and postoperative damage, and preferably the organ that is to be protected does not experience one or more (including all) of ischemia, reperfusion and thrombosis, or any other direct effect, as a result of the surgical intervention.

The peri- and postoperative complications that can be prevented and/or reduced through the uses and/or pharmaceutical compositions according to the present invention are exemplified by, but not limited to, cardiovascular complications, such as myocardial infarction, congestive heart failure, stroke and TIA (transient ischemic attack), postoperative limb ischemia, kidney complications, such as renal dysfunction and nephritis, liver injury, gastrointestinal complications such as ischaemic intestinal disease, pulmonary complication such as pulmonary embolism.

Depending on the nature and location of the surgical intervention, the organ in which the peri- or postoperative complication is prevented and/or reduced in accordance with in an embodiment of the first aspect of the present invention may be selected from any one or more of the following organs -
- One or more organs in the abdomen or pelvis, such as adrenals; appendix; bladder; gallbladder; large intestine; small intestine; kidney; liver; pancreas; spleen; stomach; in the male pelvis the prostate and/or testes; in the female pelvis the ovaries and/or uterus;
- One or more organs in the thorax, such as heart; lung; esophagus; thymus; pleura;
- One or more organs in the head and/or neck, such as the brain (including brain basal ganglia; brain stem or part thereof e.g. medulla, midbrain, pons; cerebellum; cerebral cortex; hypothalamus; limbic system or part thereof e.g. amygdale);
- Eye;
- Pituitary;
- Thyroid and Parathyroids.

Peri- and post operative complications are well known to the skilled person and well characterised in the art. A peri- or postoperative complication that can be treated or prevented in accordance with the present invention may, for example, include a partial or total loss of the measurable function of one or more organs. Means and methods for the measurement of organ function will vary from organ to organ, and are well known to those skilled in the art. Organ function, or organ damage can, for example, be established by the measurement of art-known markers for the function or damage of an organ of interest in samples obtained from the patient. For example, plasma levels of lactate dehydrogenase (LDH) and myoglobin may be used as a measurement of target organ injury; levels of aspartic acid aminotransferase (ASAT) are a suitable marker for liver injury (such as during ischemia and reperfusion); serum levels of cardiac specific troponins or ECG for cardiac injury; urine protein levels for the measurement of kidney injury; and white blood cell number in the lungs or functional lung capacity tests can be used to measure acute lung injury (such as following intestinal ischemia).

In a pharmaceutical composition and use according to the first aspect of the invention that is intended to prevent or reduce peri- and postoperative complications, the Annexin A5 or functional analogue or variant thereof is typically administered at the time of or shortly before the surgical intervention. For example, it may be administered at least 1 week, 6 days, 5 days, 4 days, 3 days, 2 days, 1 day, 12 hours, 8 hours, 4 hours, 2 hours, 1 hour, or 50, 40, 30, 20, 10, 5, 4 ,3, 2, 1, or less than 1 minutes before the surgical intervention or at the time of surgical the intervention. Typically, administration of the Annexin A5 or functional analogue or variant thereof is continued after the surgical intervention, for example for at least 1 week, 2 weeks, 1 month, 2 months, 6 months, 1 year, 2 years, 5 years or 10 years after the intervention. Annexin A5 or functional analogue or variant thereof may be provided by repeated administration of suitable dosage forms, or by administration in a controlled release form, or as a combination of one or more forms of administration. It will be appreciated that the exact duration of continuation of administration of the Annexin A5 or functional analogue or variant thereof may be chosen to coincide with the degree of risk of peri- and postoperative complications at different times after the surgical intervention.

In other embodiments of the first aspect of the invention, Annexin A5 or functional analogue or variant thereof is administered as a secondary prophylaxis, to prevent or reduce worsening of peri- and postoperative complications that has already occurred; or as a treatment, to completely or partially reverse peri- and postoperative complications that have already occurred.

Typically, Annexin A5 or functional analogue or variant thereof is provided by repeat administration of suitable dosage forms. Typically, administration is continued for at least 1 week, 2 weeks, 1 month, 2 months, 6 months, 1 year, 2 years, 5 years or 10 years after the surgical intervention

By administering a "therapeutically effective amount of Annexin A5 or a functional analogue or variant thereof", we mean administering an amount which has a beneficial effect in preventing and/or reducing peri- and postoperative complications, or preventing and/or reducing the worsening of existing peri- and postoperative complications. Where the Annexin A5 or a functional analogue or variant thereof prevents and/or reduces peri- and postoperative complications typically, peri- and postoperative complications are not seen following onset of administration, or are seen later than would be expected (i.e. compared to its development in the absence of the administration of Annexin A5 or functional analogue or variant thereof), or develops less quickly, or develops to a reduced extent, or there is a combination of slower progression and reduced development.

Typically, postoperative complications would be seen at least, 1 hour, 6 hours, 12 hours, 1 day, 1 week, 2 weeks, 1 month, at least 3 months, 6 months, 1 year, 2 years, 5 years or 10 years later than would have been expected in the absence of administration of Annexin A5 or a functional analogue or variant thereof. Typically, where the Annexin A5 or a functional analogue or variant thereof is administered to prevent and/or reduce existing postoperative complications, symptoms of postoperative complications disappear over time, such that postoperative complications is no longer seen at 10 years, at 5 years, 2 years, 1 year, 6 months, 3 months, 1 month, 2 weeks, 1 week, 12 hours, 6 hours following administration.

The skilled person will appreciate that different endpoints may be appropriate to identify a therapeutic effect of administration of Annexin A5 or functional analogue or variant thereof. For example, myocardial infarction and/or death are common postoperative complications following peripheral vascular surgery. Subjects administered Annexin A5 or functional analogue or variant thereof may be afflicted by myocardial infarction at lower incidence, and/or survive for longer time than would be expected in the absence of the administration of Annexin A5 or functional analogue or variant thereof.

A further demonstration of the effect of administration of Annexin A5 or a functional analogue or variant thereof on postoperative complications following surgical interventions can be determined by clinical trials, typically involving tens, hundreds, or thousands of patients (such as 10, 50, 100, 1000 or 10000 or values between these). Alternatively, animal tests may be used, such as those described below in relation to the testing of functional analogues and variants of Annexin A5, and in the Examples.

The Annexin A5 or the functional analogue or variant thereof can be administered in conjunction with one or more further active agent(s), such as a thrombolytic therapeutic such as aspirin, clopidogrel, ticlopidin, tissue plasminogen activator, urokinase, or a bacterial enzyme such as streptokinase; an analgesic therapeutic such as an opiate, an anti-infective therapeutic such as a beta-lactam, a tetracycline, an amphenicol, or an aminoglycoside. The Annexin A5 or functional analogue or variant thereof can be co-administered with any of one or more further active agent(s), or it may be administered separately, simultaneously or sequentially with such agent(s). Typically, one or more of these agents is used to reduce the risk of thrombosis, which can occlude blood vessels, including stented blood vessels. Suitably, therapy is commenced with ticlopidin or clopidogrel before a revascularisation procedure involving stent deployment, and is continued for at least three, six or even twelve months after the procedure (Ong and Serruys, 2005). Typically, aspirin is administered concomitantly with ticlopidin or clopidogrel and then continued indefinitely. The use of aspirin together with clopidogrel or ticlopidin is referred to as dual antiplatelet therapy. Similar regimes of antithrombotic drug administration may be applied before and following other types of intervention.

The Annexin A5 or the functional analogue or variant thereof can be administered parenterally, intravenously, intra-arterially, intra-peritoneally, intra-muscularly, or subcutaneously or locally.

A pharmaceutical composition according to the first aspect of the invention may comprise Annexin A5 or a functional analogue or variant thereof in admixture with a pharmaceutically or veterinarily acceptable adjuvant, diluent or carrier, which will typically be selected with regard to the intended route of administration and standard pharmaceutical practice. The composition may be in the form of immediate-, delayed- or controlled-release applications. Preferably, the formulation is a unit dosage containing a daily dose or unit, daily sub-dose or an appropriate fraction thereof, of the active ingredient.

The phrases "pharmaceutical or pharmacologically acceptable" refer to compositions that do not produce an adverse, allergic or other untoward reaction when administered to an animal, such as, for example, a human, as appropriate. The preparation of such pharmaceutical compositions are known to those of skill in the art in light of the present disclosure, as exemplified by Remington's Pharmaceutical Sciences, 18th Ed. Mack Printing Company, 1990. Moreover, for animal (*e.g*., human) administration, it will be understood that preparations should meet sterility, pyrogenicity, general safety and purity standards as required by FDA Office of Biological Standards.

As used herein, "pharmaceutically acceptable carrier" includes any and all solvents, dispersion media, coatings, surfactants, antioxidants, preservatives (*e.g*., antibacterial agents, antifungal agents), isotonic agents, salts, preservatives, drugs, drug stabilizers, excipients, disintegration agents, such like materials and combinations thereof, as would be known to one of ordinary skill in the art. Except insofar as any conventional carrier is incompatible with the active ingredient, its use in the therapeutic or pharmaceutical compositions is contemplated.

The pharmaceutical compositions according to the invention may, or may not, be intended for, and, thus formulated in a manner suitable for, parenteral, intravenous, intra-arterial, intraperitoneal, intra-muscular or subcutaneous administration, or administration from a drug-eluting stent, or they may be administered by infusion techniques. Sterile injectable solutions may be prepared by incorporating the active compounds in the required amount in the appropriate solvent with various of the other ingredients enumerated above, as required, followed by sterilization. The pharmaceutical compositions may be best used in the form of a sterile aqueous solution which may contain other substances, for example, enough salts or glucose to make the solution isotonic with blood. The aqueous solutions may be suitably buffered (preferably to a pH of from 3 to 9), if necessary. The preparation of suitable pharmaceutical formulations under sterile conditions is readily accomplished by standard pharmaceutical techniques well-known to those skilled in the art.

A therapeutically effective amount of Annexin A5 or a functional analogue or variant thereof for administration to a patient, such as a human patient, on the basis of a daily dosage level may be from 0.01 to 1000 mg of Annexin A5 or a functional analogue or variant thereof per adult (for example, from about 0.001 to 20 mg per kg of the patient's body weight, such as 0.01 to 10 mg/kg, for example greater than 0.1 mg/kg and up to or less than 20, 10, 5, 4, 3 or 2 mg/kg, such as about 1 mg/kg), administered in single or divided doses.

The physician in any event will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention.

For veterinary use, a compound of the invention is administered as a suitably acceptable formulation in accordance with normal veterinary practice and the veterinary surgeon will determine the dosing regimen and route of administration which will be most appropriate for a particular animal.

The Annexin A5 or functional analogue or variant thereof referred to in relation to the first aspect of the invention may be selected from:
a) human Annexin A5 (SEQ ID NO:1);
b) a mammalian orthologue of human Annexin A5;
c) an allelic or genetic variant of a) or b);
d) a functional analogue of Annexin which is a protein which is more than 90%, or preferably more than 95% or 99% identical to human Annexin A5, SEQ ID NO:1;
e) a dimer of, or fusion protein comprising, any of a), b), c) or d); and
f) a PEGylated variant of any of a), b), c), d) or e).

In particular embodiments, the functional analogue or variant of Annexin A5 according to the invention is more than 90%, or preferably more than 95% or 99% identical to human Annexin A5, SEQ ID NO:1.

The percent identity between two amino acid sequences is determined as follows. First, an amino acid sequence is compared to, for example, SEQ ID NO:1 using the BLAST 2 Sequences (Bl2seq) program from the stand-alone version of BLASTZ containing BLASTN version 2.0.14 and BLASTP version 2.0.14. This stand-alone version of BLASTZ can be obtained from the U.S. government's National Center for Biotechnology Information web site at ncbi.nlm.nih.gov. Instructions explaining how to use the Bl2seq program can be found in the readme file accompanying BLASTZ. Bl2seq performs a comparison between two amino acid sequences using the BLASTP algorithm. To compare two amino acid sequences, the options of Bl2seq are set as follows: -i is set to a file containing the first amino acid sequence to be compared (e.g., C:\seq1.txt); -j is set to a file containing the second amino acid sequence to be compared (e.g., C:\seq2.txt); - p is set to blastp; -o is set to any desired file name (e.g., C:\output.txt); and all other options are left at their default setting. For example, the following command can be used to generate an output file containing a comparison between two amino acid sequences: C:\Bl2seq -i c:\seq1.txt -j c:\seq2.txt -p blastp -o c:\output.txt. If the two compared sequences share homology, then the designated output file will present those regions of homology as aligned sequences. If the two compared sequences do not share homology, then the designated output file will not present aligned sequences. Once aligned, the number of matches is determined by counting the number of positions where an identical nucleotide or amino acid residue is presented in both sequences.

The percent identity is determined by dividing the number of matches by the length of the sequence set forth in an identified sequence followed by multiplying the resulting value by 100. For example, if a sequence is compared to the sequence set forth in SEQ ID NO:1 (the length of the sequence set forth in SEQ ID NO:1 is 320) and the number of matches is 288, then the sequence has a percent identity of 90 (*i.e.,* 288 = 320 * 100 = 90) to the sequence set forth in SEQ ID NO:1.

Thus, a functional analogue or variant of Annexin A5 as defined by Claim 1 and Claim 2 may be a protein wherein at one or more positions there have been amino acid insertions, deletions, or substitutions, either conservative or non-conservative, provided that such changes result in a protein whose basic properties to function in an equivalent manner to Annexin A5 have not significantly been changed. "Significantly" in this context means that one skilled in the art would say that the properties of the variant may still be different but would not be unobvious over the ones of the original protein.

By "conservative substitutions" is intended combinations such as Gly, Ala; Val, Ile, Leu; Asp, Glu; Asn, Gin; Ser, Thr; Lys, Arg; and Phe, Tyr.

Such variants may be made using the methods of protein engineering and site-directed mutagenesis which are well known in the art.

The functional analogue or variant of Annexin A5 according to the invention may be a dimer of Annexin A5 (such as DiAnnexin) or a functional analogue or variant thereof, or may be a PEGylated Annexin A5 or a functional analogue or variant thereof. DiAnnexinA5 and PEGylated AnnexinA5 are disclosed in WO 02/067857.

PEGylation is a method well known to those skilled in the art wherein a polypeptide or peptidomimetic compound (for the purposes of the present invention, Annexin A5 or the functional analogue or variant) is modified such that one or more polyethylene glycol (PEG) molecules are covalently attached to the side chain of one or more amino acids or derivatives thereof. It is one of the most important molecule altering structural chemistry techniques (MASC). Other MASC techniques may be used; such techniques may improve the pharmacodynamic properties of the molecule, for example extending its half-life *in vivo.* A PEG-protein conjugate is formed by first activating the PEG moiety so that it will react with, and couple to, the protein or peptidomimetic compound of the invention. PEG moieties vary considerably in molecular weight and conformation, with the early moieties (monofunctional PEGs; mPEGs) being linear with molecular weights of 12kDa or less, and later moieties being of increased molecular weights. PEG2, a recent innovation in PEG technology, involves the coupling of a 30kDa (or less) mPEG to a lysine amino acid (although PEGylation can be extended to the addition of PEG to other amino acids) that is further reacted to form a branched structure that behaves like a linear mPEG of much greater molecular weight (Kozlowski et al., 2001). Methods that may be used to covalently attach the PEG molecules to polypeptides are further described in Roberts et al. (2002) Adv Drug Deliv Rev, 54, 459 - 476; Bhadra et al. (2002) Pharmazie 57, 5 - 29; Kozlowski et al. (2001) J Control Release 72, 217 - 224; and Veronese (2001) Biomaterials 22, 405 - 417 and references referred to therein.

The advantages of PEGylation include reduced renal clearance which, for some products, results in a more sustained adsorption after administration as well as restricted distribution, possibly leading to a more constant and sustained plasma concentrations and hence an increase in clinical effectiveness (Harris et al. (2001) Clin Pharmacokinet 40, 539 - 551). Further advantages can include reduced immunogenicity of the therapeutic compound (Reddy (2001) Ann Pharmacother, 34, 915 - 923), and lower toxicity (Kozlowski et al. (2001), Biodrugs 15, 419 - 429).

The functional analogue or variant of Annexin A5 can be a fusion protein comprising the sequence of Annexin A5 or a variant thereof. Thus, for example, Annexin A5 or a variant thereof can be fused to one or more fusion partner polypeptide sequence(s) so as to extend the half-life of the molecule within a patient's circulatory system and/or add further functionality to the molecule.

A "functional" analogue or variant of Annexin A5 may be capable of binding to phosphatidylserine on a biological membrane, preferably to a level that is at least 10%, 20%, 30%, 40%, 50%, 60%, 70%, 80%, 90%, 95%, 99% or about 100% of that displayed by human Annexin A5 (SEQ ID NO:1) under the same conditions. A suitable method for measuring Annexin A5 binding to phosphatidylserine on a biological membrane is known in the art (Vermes et al. (1995) J Immunol Methods, 184(1): p. 39-51).

A "functional" analogue or variant of Annexin A5 may, additionally, or alternatively, possess at least 10%, 20%, 30%, 40%, 50%, 60%, 70%, 80%, 90%, 95%, 99% or about 100% of the therapeutic activity human Annexin A5 (SEQ ID NO:1) when used at the same (i.e. molar equivalent) dosage, for prophylaxis or treatment of restenosis in accordance with the first aspect of the invention. In this context, the therapeutic activity of a "functional" analogue or variant of Annexin A5 may be determined, compared to that of human Annexin A5 (SEQ ID NO:1), by comparing the ability of a molar equivalent amount of the functional analogue or variant and of human Annexin A5 to treat or provide prophylaxis for restenosis as described above, and/or by animal tests, such as using the methodology set out in the following examples.

### Examples

The following examples are included to demonstrate particular embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

### Example 1: Rat hind limb surgery model

The aim of the experiments described in this example is to perform a surgical intervention in rats in a peripheral organ to which blood flow is stopped during the surgery.

For this purpose, rats are anaesthetised and a ligature is placed around the lower abdominal aorta. The threads are passed through a PE50 catheter, and the blood flow is occluded by pushing the threads up through the catheter so the aorta becomes clamped, and blood flow to the hind limbs is stopped. The suture is then fixed in the closed position by clamping the catheter. In this experiment, there is no principal intervention made, but during the occlusion period, the left femoral artery is exposed and gently clamped, to mimic a trauma obtained during surgery. Forty-five minutes after the stopping of blood flow, the aorta is again opened and blood flow re-instituted.

This experimental protocol is performed as an example of a reproducible surgical intervention. Blood samples are taken from an indwelling catheter before blood flow to the hind limbs are stopped, and 180 minutes after blood flow is re-instituted. Rats are administered intravenously with Annexin A5 in a dose of 1 mg/kg or placebo at least 10 minutes prior to anaesthesia.

Plasma levels of lactate dehydrogenase (LDH) and myoglobin are used as measured as of target organ injury. These proteins are intracellular, but are released from muscle cells that are injured. It is also well known that myoglobin is a common case of renal failure, so the level of myoglobin is also a measure of the risk for a complication in a non-target organ.

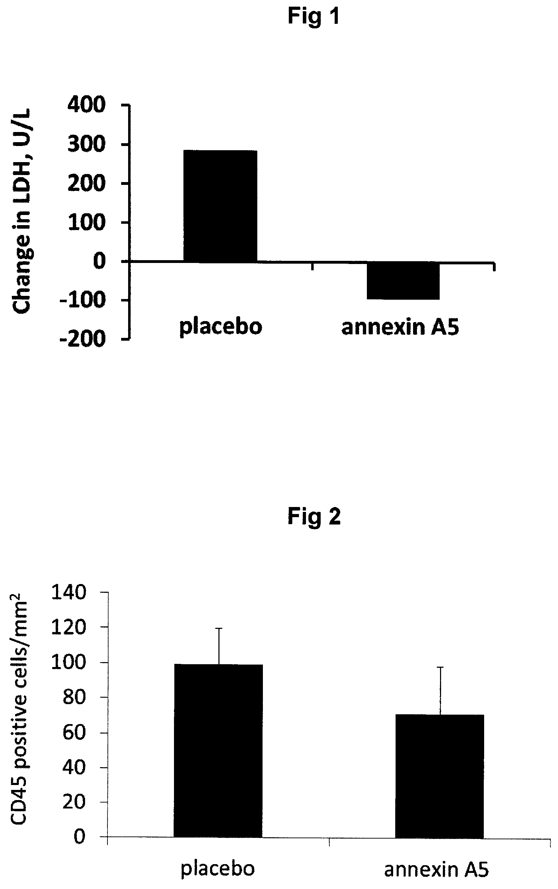

In all animals, plasma levels were quite high already at time 0, most likely reflecting that anaesthesia and surgery itself is traumatic and associated to tissue injury. In sham-operated animals (that is animals that had the ligature placed around the aorta, but they were not subjected to ischemia) there was a marked drop during the experimental period. However, as is seen in Figure 1, in untreated (placebo) animals the plasma LDH levels continued to increase, whereas if the animals were administered annexin A5 prior to ischemia, there was instead a slight reduction in LDH levels.

Interestingly, we also found an increase in aspartic acid aminotransferase (ASAT), a marker for liver injury during ischemia and reperfusion (but not in the sham operated rats), indicating that the procedure caused liver injury. There was an increase in both placebo and annexin A5 treated rats, but annexin A5 treatment reduced the increase. Further, major increase in myoglobin levels were found in 25 % of the placebo treated rats, but in no annexin A5 treated rat.

This experiment shows that Annexin A5 can limit peri- and postoperative complications.

### Example 2.

Intestinal ischemia/reperfusion (I/R) injury is a grave condition resulting from acute mesenteric ischemia, haemorrhagic, traumatic or septic shock, or severe burns and some surgical procedures including small bowel transplantation and abdominal aortic surgery . Multiple organ failure, including acute lung injury (ALI) and its severe form acute respiratory distress syndrome (ARDS), is a common complication of intestinal I/R injuries and contributes to its high mortality rate (Ding R et al (2012), Inflammation 35(1):158-66).

This example tests the hypothesis that Annexin A5 can prevent or reduce the injury in lungs following intestinal surgery. Acute lung injury was induced by intestinal ischemia-reperfusion. Briefly, Sprague-Dawley rats (approximately 250 g) were anesthetized, and a midline laparatomy performed and the superior mesenteric artery identified and occluded below the celiac trunk with an arterial microclamp. Intestinal ischemia is confirmed by paleness of the jejunum and ileum. After 45 min of ischemia, reperfusion is initiated by removal of the clamp and confirmed by the colour recovery of the intestine. Pre-warmed (37°C) saline (0.5 ml) is instilled into the peritoneal cavity before closed with a suture. The animals are sacrificed 2 hours later by exsanguinations. The rats are administered intravenously with 1 mg/kg of annexin A5 or placebo one hour before ischemia.

At termination the lung tissue was isolated and fixed in formalin solution for further processing and histopathological analyses All samples were processed from formalin through graded alcohols, xylene to paraffin wax and subsequently embedded in paraffin wax. 5µm sections were cut from sample and placed onto Superfrost Plus slides. They were stained to demonstrate white blood cells and lymphocytes using an antibody, CD45, in an immunohistochemical (IHC) assay and subsequently digitally scanned by OracleBio. The scanned image was then analysed using Definien's Tissue Studio 3.0 software. Image analysis showed that annexin A5 treatment reduced the number of white cells (figure 2), showing that annexin A5 reduced acute lung injury following intestinal ischemia.

### References

The following references, to the extent that they provide exemplary procedural or other details supplementary to those set forth herein, are mentioned:
PCT Appln. WO 02/067857
PCT Appln. WO 2005/099744
PCT Appln WO 2009/077764)
PCT Appln WO 2009/103977.
Bhadra et al. (2002) Pharmazie 57, 5 - 29
Cederholm and Frostegard (2007) Ann. NY Acad. Sci., 1108:96-103.
Cederholm et al. (2005) Arterioscler. Thromb. Vasc. Biol., 25(1):198-203.
Ding R et al (2012), Inflammation 35(1):158-66
Duggan. Am J Cardiovasc Drugs, 2012. 12(1): p. 57-72.
Feinman. Am J Physiol Gastrointest Liver Physiol, 2010. 299(4): p. G833-43.
Harris et al. (2001) Clin Pharmacokinet 40, 539 - 551
Kozlowski et al. (2001) J Control Release 72, 217 - 224
Kozlowski et al. (2001), Biodrugs 15, 419 - 429
Ong and Serruys (2005) Curr Issues Cardiol 32: 372-377
Rand (2000) Autoimmun., 15(2):107-111.
Reddy (2001) Ann Pharmacother, 34, 915 - 923
Roberts et al. (2002) Adv Drug Deliv Rev, 54, 459 - 476
Shen et al. (2007) Am. J. Transplant., 7(11):2463-2471.
Smith Jr. et al. (2006) J. Am. Coll. Cardiol., 47(1):216-235.
Teoh et al. (2007) Gastroenterology, 133(2):632-646.
Thiagarajan and Benedict (1997) Circulation, 96(7):2339-2347.
Vermes et al. (1995) J Immunol Methods, 184(1): p. 39-51
Veronese (2001) Biomaterials 22, 405 - 417
Vogt et al. (2004) European Heart Journal 25: 1330-40.
Zayour and Azar. Endocr Pract, 2006. 12(1): p. 59-62.
Zhang et al. (2004) Arterioscler. Thromb. Vasc. Biol., 24(12):2277-2283.

## Claims

1. A pharmaceutical composition comprising a therapeutically effective amount of Annexin A5 or a functional analogue or variant thereof for use in the prevention and/or reduction of peri- and postoperative complications following surgical intervention,
wherein the surgical intervention comprises restrictions in organ blood flow during the surgical intervention, and further wherein the surgical intervention causes immediate cellular damage, ischemia and/or reperfusion injury to a target organ,
wherein the peri- or postoperative complication is a complication of an organ not being the target of the surgical intervention, and
wherein the Annexin A5 or the functional analogue or variant thereof is selected from:
(a) human Annexin A5 (SEQ ID NO:1);
(b) a mammalian orthologue of human Annexin A5;
(c) an allelic or genetic variant of a) or b);
(d) a functional analogue of Annexin which is a protein which is more than 90%, or preferably more than 95% identical to human Annexin A5, SEQ ID NO:1;
(e) a dimer of, or a fusion protein comprising, any of a), b), c) or d); and
(f) a PEGylated variant of any of a), b), c), d) or e).

2. Annexin A5 or a functional analogue or variant thereof for use in the prevention and/or reduction of peri- and postoperative complications following surgical intervention,
wherein the surgical intervention comprises restrictions in organ blood flow during the surgical intervention, and further wherein the surgical intervention causes immediate cellular damage, ischemia and/or reperfusion injury to a target organ,
wherein the peri- or postoperative complication is a complication of an organ not being the target of the surgical intervention, and
wherein the Annexin A5 or the functional analogue or variant thereof is selected from:
(a) human Annexin A5 (SEQ ID NO:1);
(b) a mammalian orthologue of human Annexin A5;
(c) an allelic or genetic variant of a) or b);
(d) a functional analogue of Annexin which is a protein which is more than 90%, or preferably more than 95% identical to human Annexin A5, SEQ ID NO:1;
(e) a dimer of, or a fusion protein comprising, any of a), b), c) or d); and
(f) a PEGylated variant of any of a), b), c), d) or e).

3. The pharmaceutical composition for use according to Claim 1, or the product for use according to Claim 2, wherein the surgical intervention comprises orthopaedic surgery, such as joint replacement.

4. The pharmaceutical composition for use according to Claim 1, or the product for use according to Claim 2, wherein the surgical intervention comprises peripheral vascular surgery.

5. The pharmaceutical composition for use according to Claim 1, or the product for use according to Claim 2, wherein the surgical intervention comprises cardiac surgery.

6. The pharmaceutical composition for use according to any of Claims 1 or 3 to 5, or the product for use according to any of Claims 2 to 5, wherein the surgical intervention does not cause immediate cellular damage, ischemia and/or reperfusion injury to, and/or thrombosis in, the organ in which the peri- or postoperative complication is prevented and/or reduced.

7. The pharmaceutical composition for use according to any of Claims 1 or 3 to 6, or the product for use according to any of Claims 2 to 6, wherein the peri- or postoperative complication is a complication is selected from a renal complication, a pulmonary complication, a gastrointestinal complication.

8. The pharmaceutical composition for use according to any of Claims 1 or 3 to 6, or the product for use according to any of Claims 2 to 6, wherein the peri- or postoperative complication is a cardiovascular complication.

9. The pharmaceutical composition for use, or the product for use, according to Claim 8, wherein the peri- or postoperative complication is selected from myocardial infarction, congestive heart disease, stroke, TIA, and postoperative limb ischemia.

10. The pharmaceutical composition for use according to any of Claims 1 or 3 to 9, or the product for use according to any of Claims 2 to 9, intended for parenteral, intravenous, intra-arterial, intra-peritoneal, intra-muscular or subcutaneous administration, or local administration from a drug eluting stent.

## Patentansprüche

1. Pharmazeutische Zusammensetzung, umfassend eine therapeutisch wirksame Menge von Annexin A5 oder einem funktionellen Analogon davon oder einer Variante davon für den Gebrauch beim Vorbeugen und/oder Verringern von peri- oder postoperativen Komplikationen nach einem chirurgischen Eingriff,
wobei der chirurgische Eingriff Einschränkungen von Organdurchblutung während des chirurgischen Eingriffs umfasst und ferner wobei der chirurgische Eingriff unmittelbare Zellschäden, Ischämie und/oder Reperfusionsschaden an einem Zielorgan verursacht,
wobei die peri- oder postoperative Komplikation eine Komplikation an einem Organ ist, das nicht das Ziel des chirurgischen Eingriffs ist, und
wobei das Annexin A5 oder das funktionelle Analogon davon oder die Variante davon ausgewählt ist aus:
(a) humanem Annexin A5 (SEQ ID NO:1);
(b) einem Säuger-Orthologon von humanem Annexin A5;
(c) einer allelischen oder einer genetischen Variante von a) oder b);
(d) einem funktionellen Analogon von Annexin, welches ein Protein ist, das zu über 90 % oder
vorzugweise über 95 % mit humanem Annexin A5, SEQ ID NO:1 identisch ist
(e) einem Dimer aus beliebigen oder einem Fusionsprotein umfassend beliebige aus a), b), c) und d); und
(f) einer PEGylierten Variante von beliebigen aus a), b), c), d) und e).

2. Annexin A5 oder ein funktionelles Analogon davon oder eine Variante davon für den Gebrauch beim Vorbeugen und/oder Verringern von peri- oder postoperativen Komplikationen nach einem chirurgischen Eingriff,
wobei der chirurgische Eingriff Einschränkungen von Organdurchblutung während des chirurgischen Eingriffs umfasst und ferner wobei der chirurgische Eingriff unmittelbare Zellschäden, Ischämie und/oder Reperfusionsschaden an einem Zielorgan verursacht,
wobei die peri- oder postoperative Komplikation eine Komplikation an einem Organ ist, das nicht das Ziel des chirurgischen Eingriffs ist, und
wobei das Annexin A5 oder das funktionelle Analogon davon oder die Variante davon ausgewählt ist aus:
(a) humanem Annexin A5 (SEQ ID NO:1);
(b) einem Säuger-Orthologon von humanem Annexin A5;
(c) einer allelischen oder einer genetischen Variante von a) oder b);
(d) einem funktionellen Analogon von Annexin, welches ein Protein ist, das zu über 90 % oder
vorzugweise über 95 % mit humanem Annexin A5, SEQ ID NO:1 identisch ist
(e) einem Dimer oder einem Fusionsprotein, umfassend beliebige aus a), b), c) und d); und
(f) einer PEGylierten Variante von beliebigen aus a), b), c), d) und e).

3. Pharmazeutische Zusammensetzung für den Gebrauch nach Anspruch 1 oder Produkt für den Gebrauch nach Anspruch 2, wobei der chirurgische Eingriff orthopädische Chirurgie, wie etwa Gelenkersatz umfasst.

4. Pharmazeutische Zusammensetzung für den Gebrauch nach Anspruch 1 oder Produkt für den Gebrauch nach Anspruch 2, wobei der chirurgische Eingriff periphere Gefäßchirurgie umfasst.

5. Pharmazeutische Zusammensetzung für den Gebrauch nach Anspruch 1 oder Produkt für den Gebrauch nach Anspruch 2, wobei der chirurgische Eingriff Herzchirurgie umfasst.

6. Pharmazeutische Zusammensetzung für den Gebrauch nach einem der Ansprüche 1 oder 3 bis 5 oder Produkt für den Gebrauch nach einem der Ansprüche 2 bis 5, wobei der chirurgische Eingriff nicht unmittelbare Zellschäden, Ischämie und/oder Reperfusionsschaden an und/oder Thrombose in dem Organ verursacht, in dem die peri- oder postoperative Komplikation verhindert und/oder verringert wird.

7. Pharmazeutische Zusammensetzung für den Gebrauch nach einem der Ansprüche 1 oder 3 bis 6 oder Produkt für den Gebrauch nach einem der Ansprüche 2 bis 6, wobei die peri- oder postoperative Komplikation eine Komplikation ist, ausgewählt aus einer Nierenkomplikation, einer Lungenkomplikation und einer gastrointestinalen Komplikation.

8. Pharmazeutische Zusammensetzung für den Gebrauch nach einem der Ansprüche 1 oder 3 bis 6 oder Produkt für den Gebrauch nach einem der Ansprüche 2 bis 6, wobei die peri- oder postoperative Komplikation eine kardiovaskuläre Komplikation ist.

9. Pharmazeutische Zusammensetzung für den Gebrauch oder Produkt für den Gebrauch nach Anspruch 8, wobei die peri- oder postoperative Komplikation ausgewählt ist aus Myokardinfarkt, kongestiver Herzinsuffizienz, Schlaganfall, TIA und postoperativer Extremitätenischämie.

10. Pharmazeutische Zusammensetzung für den Gebrauch nach einem der Ansprüche 1 oder 3 bis 9 oder Produkt für den Gebrauch nach einem der Ansprüche 2 bis 9, vorgesehen für parenterales, intravenöses, intraarterielles, intraperitoneales, intramuskuläres oder subkutanes Verabreichen oder für lokales Verabreichen über einen mit Medikamenten beschichteten Stent.

## Revendications

1. Composition pharmaceutique comprenant une quantité thérapeutiquement efficace d'Annexine A5 ou d'un analogue ou d'un variant fonctionnel de celle-ci, pour une utilisation dans la prévention et/ou la réduction de complications péri- et postopératoires après une intervention chirurgicale,
dans laquelle l'intervention chirurgicale comprend des restrictions du flux sanguin dans les organes pendant l'intervention chirurgicale, et également dans laquelle l'intervention chirurgicale provoque un dommage cellulaire immédiat, une lésion d'ischémie et/ou de reperfusion au niveau d'un organe cible,
dans laquelle la complication péri- ou postopératoire est une complication d'un organe qui n'est pas la cible de l'intervention chirurgicale, et
dans laquelle l'Annexine A5 ou l'analogue ou le variant fonctionnel de celle-ci est choisi(e) parmi :
(a) l'Annexine A5 humaine (SEQ ID n° : 1);
(b) un orthologue mammifère de l'Annexine A5 humaine ;
(c) un variant allélique ou génétique de a) ou b) ;
(d) un analogue fonctionnel de l'Annexine qui est une protéine à plus de 90 %, ou de préférence à plus de 95 % identique à l'Annexine A5 humaine, SEQ ID n° : 1 ;
(e) un dimère de, ou une protéine de fusion comprenant, l'un quelconque de a), b), c) ou d) ; et
(f) un variant pégylé de l'un quelconque de a), b), c), d) ou e).

2. Annexine A5 ou analogue ou variant fonctionnel de celle-ci, pour une utilisation dans la prévention et/ou la réduction de complications péri- et postopératoires après une intervention chirurgicale,
dans laquelle l'intervention chirurgicale comprend des restrictions du flux sanguin dans les organes pendant l'intervention chirurgicale, et également dans laquelle l'intervention chirurgicale provoque un dommage cellulaire immédiat, une lésion d'ischémie et/ou de reperfusion au niveau d'un organe cible,
dans laquelle la complication péri- ou postopératoire est une complication d'un organe qui n'est pas la cible de l'intervention chirurgicale, et
dans laquelle l'Annexine A5 ou l'analogue ou le variant fonctionnel de celle-ci est choisi(e) parmi :
(a) l'Annexine A5 humaine (SEQ ID n° : 1);
(b) un orthologue mammifère de l'Annexine A5 humaine;
(c) un variant allélique ou génétique de a) ou b) ;
(d) un analogue fonctionnel de l'Annexine qui est une protéine à plus de 90 %, ou de préférence à plus de 95 % identique à l'Annexine A5 humaine, SEQ ID n° : 1 ;
(e) un dimère de, ou une protéine de fusion comprenant, l'un quelconque de a), b), c) ou d) ; et
(f) un variant pégylé de l'un quelconque de a), b), c), d) ou e).

3. Composition pharmaceutique pour une utilisation selon la revendication 1, ou produit pour une utilisation selon la revendication 2, dans laquelle ou dans lequel l'intervention chirurgicale comprend la chirurgie orthopédique telle que le remplacement articulaire.

4. Composition pharmaceutique pour une utilisation selon la revendication 1, ou produit pour une utilisation selon la revendication 2, dans laquelle ou dans lequel l'intervention chirurgicale comprend la chirurgie vasculaire périphérique.

5. Composition pharmaceutique pour une utilisation selon la revendication 1, ou produit pour une utilisation selon la revendication 2, dans laquelle ou dans lequel l'intervention chirurgicale comprend la chirurgie cardiaque.

6. Composition pharmaceutique pour une utilisation selon l'une quelconque des revendications 1 ou 3 à 5, ou produit pour une utilisation selon l'une quelconque des revendications 2 à 5, dans laquelle ou dans lequel l'intervention chirurgicale ne provoque pas un dommage cellulaire immédiat, une lésion d'ischémie et/ou de reperfusion au niveau de, et/ou une thrombose dans, l'organe dans lequel la complication péri- ou postopératoire est prévenue et/ou réduite.

7. Composition pharmaceutique pour une utilisation selon l'une quelconque des revendications 1 ou 3 à 6, ou produit pour une utilisation selon l'une quelconque des revendications 2 à 6, dans laquelle ou dans lequel la complication péri- ou postopératoire est une complication choisie parmi une complication rénale, une complication pulmonaire, une complication gastro-intestinale.

8. Composition pharmaceutique pour une utilisation selon l'une quelconque des revendications 1 ou 3 à 6, ou produit pour une utilisation selon l'une quelconque des revendications 2 à 6, dans laquelle ou dans lequel la complication péri- ou postopératoire est une complication cardiovasculaire.

9. Composition pharmaceutique pour une utilisation, ou produit pour une utilisation, selon la revendication 8, dans laquelle ou dans lequel la complication péri- ou postopératoire est choisie parmi l'infarctus du myocarde, l'insuffisance cardiaque congestive, l'accident vasculaire cérébral, l'AIT et l'ischémie post-opératoire des membres.

10. Composition pharmaceutique pour une utilisation selon l'une quelconque des revendications 1 ou 3 à 9, ou produit pour une utilisation selon l'une quelconque des revendications 2 à 9, destiné(e) à une administration par voie parentérale, intraveineuse, intra-artérielle, intra-péritonéale, intramusculaire ou sous-cutanée ou à une administration locale depuis une endoprothèse à élution médicamenteuse.
